# EUROPÄISCHE PATENTANMELDUNG

(11) **EP 2 116 211 A1**
(43) Veröffentlichungstag der Anmeldung: **11.11.2009**
(21) Anmeldenummer: 08008423.9
(22) Anmeldetag: 05.05.2008
(51) Int. Cl.: A61F 2/44

(54) **Zwischenwirbelprothese**

(71) Anmelder: Röbling, Christian, 79104 Freiburg (DE); Ackermann, Rolf, 78604 Rietheim-Weilheim (DE)
(72) Erfinder: Röbling, Christian, 79104 Freiburg (DE); Ackermann, Rolf, 78604 Rietheim-Weilheim (DE)
(74) Vertreter: Modrow, Stephanie

(57) **Zusammenfassung**

Die Erfindung betrifft eine Zwischenwirbelprothese (10) mit einer ersten Prothesenplatte (20) und einer zweiten Prothesenplatte (30), wobei die erste Prothesenplatte (20) auf ihrer der zweiten Prothesenplatte (30) zugewandten Seite (20b) eine konkave Ausnehmung (22) aufweist, in welche ein auf der der ersten Prothesenplatte (10) zugewandten Seite (30b) der zweiten Prothesenplatte (30) angeordneter konvexer Vorsprung (32) eingreift, wobei der konvexe Vorsprung (32) und/oder die konkave Ausnehmung zumindest ein federndes Element (35) aufweist.

## Beschreibung

Die Erfindung betrifft eine Zwischenwirbelprothese gemäß dem Oberbegriff des Patentanspruchs 1.

Bekannt sind zwei- oder mehrteilige Zwischenwirbelprothesen, wobei zwischen wenigstens zwei Teilen ein Gelenk gebildet wird, um so durch die Zwischenwirbelprothese die Bewegungsmöglichkeiten der Bandscheibe besser zu simulieren. Insbesondere bekannt sind Zwischenwirbelprothesen mit einer ersten Prothesenplatte und einer zweiten Prothesenplatte, wobei die erste Prothesenplatte auf ihrer der zweiten Prothesenplatte zugewandten Seite eine konkave Ausnehmung aufweist, in welche ein auf der der ersten Prothesenplatte zugewandten Seite der zweiten Prothesenplatte angeordneter konvexer Vorsprung eingreift, sodass auf diese Weise eine Art Kugelgelenk gebildet wird. Beim Einbringen derartiger Zwischenwirbelprothese ist in der Regel zunächst ein Aufspreizen des Zwischenraumes zwischen zwei benachbarten Wirbeln vonnöten.

Die bekannten Zwischenwirbelprothesen bestehen zumeist aus zwei Prothesenplatten aus einem Metall oder einer Prothesenplatte aus Metall und einer Prothesenplatte aus Kunststoff, wobei der Abstand zwischen den beiden Prothesenplatten definiert ist.

Die Aufgabe der Erfindung besteht darin, eine Zwischenwirbelprothese bereitzustellen, welche die zu ersetzende Bandscheibe besser simuliert.

Die Aufgabe wird erfindungsgemäß gelöst durch eine Zwischenwirbelprothese mit den Merkmalen des Anspruchs 1.

Die erfindungsgemäße Zwischenwirbelprothese mit einer ersten Prothesenplatte und einer zweiten Prothesenplatte, wobei die erste Prothesenplatte auf ihrer der zweiten Prothesenplatte zugewandten Seite eine konkave Ausnehmung aufweist, in welche ein auf der der ersten Prothesenplatte zugewandten Seite der zweiten Prothesenplatte angeordneter konvexer Vorsprung eingreift, ist derart ausgebildet, dass der konvexe Vorsprung und/oder die konkave Ausnehmung zumindest ein federndes Element aufweist. Das federnde Element erlaubt eine Relativbewegung der beiden Prothesenplatten gegeneinander in vertikaler Richtung. Das federnde Element hat den Vorteil, dass es als Dämpfung wirkt, sodass die Funktion der defekten Bandscheibe besser simuliert wird.

Besonders bevorzugt ist der konvexe Vorsprung und/oder die konkave Ausnehmung hohl ausgebildet, wodurch sich bereits eine gewisse Federwirkung oder gewisse Elastizität des konvexen Vorsprungs ergibt, der eine Relativbewegung zwischen den beiden Prothesenplatten erlaubt und eine Dämpfung bewirkt.

Besonders bevorzugt weist der konvexe Vorsprung und/oder die konkave Ausnehmung wenigstens einen Schlitz auf, wodurch insbesondere wenigstens zwei federnde Elemente gebildet werden, sodass der konvexe Vorsprung bei erhöhtem Druck tiefer in die konkave Ausnehmung der ersten Prothesenplatte hineingedrückt werden kann. Unter einem Schlitz können dabei aus Durchbrüche verstanden werden, die in zwei zueinander senkrecht stehenden Richtungen etwa die gleiche Längenausdehnung aufweisen. Zudem kann auch die Form der Schlitze beliebig variieren. Der Schlitz soll lediglich eine Federwirkung des Vorsprungs bzw. der Ausnehmung begünstigen. Die Schlitze können gerade, gekrümmt, in mehreren Krümmungen wie beispielsweise wellenförmig oder auch spiralförmig verlaufen.

Vorzugsweise sind die Schlitze schräg zur Oberfläche des konvexen Vorsprungs bzw. der konkaven Ausnehmung eingeschnitten, so dass bei hoher Belastung die Seitenränder benachbarter federnder Elemente nicht nur aneinander, sondern in radialer Richtung aufeinander zu liegen kommen, wodurch die Stabilität des Vorsprungs bzw. der Ausnehmung bei erhöht wird.

Gemäß einer besonders bevorzugten Ausführungsform der Erfindung weist der konvexe Vorsprung und/oder die konkave Ausnehmung ausgehend von seinem bzw. ihrem Pol, d. h. dem höchsten Punkt der gekrümmten Oberfläche, mehrere in Richtung des Randes des konvexen Vorsprungs verlaufende Schlitze auf. Dadurch werden mehrere federnde Elemente gebildet, die eine besonders gute Dämpfung ermöglichen.

Um die Beweglichkeit der beiden benachbarten Wirbel gegeneinander möglichst gut zu erhalten, ist vorzugsweise der Vorsprung in der Ausnehmung verdrehbar und verschwenkbar.

Der Vorsprung ist gemäß einer besonders bevorzugten Ausführung kugelsegmentförmig ausgebildet, um insbesondere ein Verdrehen und Kippen in jede beliebige Richtung zu ermöglichen. Die Ausnehmung ist vorzugsweise kugelschalensegmentförmig ausgebildet, um mit dem Vorsprung besonders gut korrespondieren zu können. Alternativ ist in einer bevorzugten Ausführungsform der Erfindung die Ausnehmung ellipsoidschalensegmentförmig ausgebildet, um zusätzlich zu der Verdrehung und Verkippung der beiden Prothesenplatten gegeneinander eine Linearbewegung, insbesondere in sagittaler Richtung, zuzulassen. Die Längsachse der ellipsoidschalensegmentförmigen Ausnehmung liegt dazu vorzugsweise in sagittaler Richtung.

Um das natürliche Rotationszentrum der Bandscheibe optimal abzubilden, ist gemäß einer bevorzugten Ausführungsform der Erfindung der Vorsprung in sagittaler Richtung von der Mitte der Prothesenplatte nach posterior versetzt angeordnet.

Gemäß einer bevorzugten Ausführungsform der Erfindung ist zwischen der ersten Prothesenplatte und der zweiten Prothesenplatte ein Führungselement angeordnet, welches einen Grundkörper und einen an einem Ende des Grundkörpers angeordneten Kopf aufweist, wobei der Grundkörper in eine Führungsausnehmung in der zweiten Prothesenplatte und der Kopf in eine Führungsausnehmung in der ersten Prothesenplatte eingreift. Der Führungsbolzen bildet einen Subluxationsschutz insbesondere bei unphysiologischen Bewegungen oder Schleudertraumen. Dazu ist das Führungselement jedoch vorzugsweise derart geführt, dass es die sonstigen Bewegungsmöglichkeiten der beiden Prothesenplatten gegeneinander kaum beeinträchtigt. Ein weiterer Vorteil eines derartigen Führungselements liegt darin, dass es als Tiefenanschlag für das federnde Element dient, um die federnde Bewegung in axialer Richtung zu begrenzen.

Vorzugsweise ist die Führungsausnehmung in der zweiten Prothesenplatte in dem Vorsprung, insbesondere zwischen den federnden Elementen, angeordnet, um so im Wesentlichen in der Nähe der Rotationsachse der Zwischenwirbelprothese zu liegen.

Bevorzugt fixiert die Führungsausnehmung der zweiten Prothesenplatte das Führungselement in horizontaler und vertikaler Richtung relativ zur zweiten Prothesenplatte. Dadurch wird eine Fixierung des Führungselements an einer Prothesenplatte erreicht.

Vorzugsweise ist die Führungsausnehmung der ersten Prothesenplatte in der konkaven Ausnehmung angeordnet, um auch in dieser Prothesenplatte die Anordnung des Führungselementes in der Nähe der Rotationsachse der Zwischenwirbelprothese zu ermöglichen.

Vorzugsweise lässt die Führungsausnehmung der ersten Prothesenplatte eine Bewegung des Führungselements in horizontaler und vertikaler Richtung zu. Insbesondere in Kombination mit der Fixierung des Führungselementes in horizontaler und vertikaler Richtung relativ zur zweiten Prothesenplatte durch die Führungsausnehmung der zweiten Prothesenplatte, schränkt das Führungselement die Relativbewegung der beiden Prothesenplatten gegeneinander kaum ein.

Um ggf. in Kombination mit einer ellipsoidschalensegmentförmigen Ausnehmung eine lineare Bewegung in sagittaler Richtung zu ermöglichen, ist vorzugsweise die Führungsausnehmung der ersten Prothesenplatte oval oder elliptisch ausgebildet, wobei die Längsachse der Führungsausnehmung in sagittaler Richtung verläuft.

Gemäß einer bevorzugten Ausnehmungsform der Erfindung sind die erste Prothesenplatte und/oder die zweite Prothesenplatte aus PEEK (Polyetheretherketon) gefertigt, welches gute biokompatible Eigenschaften aufweist. Ein besonderer Vorteil dieses Materials liegt darin, dass es sowohl CT(Computertomographie)-als auch MRT(Magnetresonanztomographie)-kompatibel ist und nicht, wie insbesondere verschiedene Metalle, Artefakte in den erstellten Bildern liefert.

Bevorzugt weist die dem Wirbel zugewandte Seite der ersten und/oder zweiten Prothesenplatte eine osteokonduktive und/oder eine osteoinduktive Beschichtung auf, um eine möglichst schnelle und dauerhafte biologische Integration der Prothese im Körper zu ermöglichen.

Gemäß einer besonders bevorzugten Ausführungsform der Erfindung ist auf der dem Wirbel zugewandten Seite wenigstens einer der Prothesenplattes wenigstens ein Verankerungselement zur Verankerung der Prothesenplatte in dem benachbarten Wirbel angeordnet ist, wobei das Verankerungselement in der Prothesenplatte versenkbar und zumindest teilweise aus der Prothesenplatte herausbewegbar angeordnet ist. Dadurch wird es ermöglicht, die Zwischenwirbelprothese mit zunächst versenkten Verankerungselementen in den Zwischenraum zwischen zwei benachbarte Wirbel einzuschieben, wobei der Zwischenraum weniger aufgespreizt werden muss, als es bei fest auf der den Wirbeln zugewandten Seite der Prothesenplatte angeordneten Verankerungselementen der Fall wäre. Ist die Zwischenwirbelprothese richtig positioniert, werden die Verankerungselemente aus der Prothesenplatte herausbewegt und stoßen somit in die benachbarten Wirbel, um die Zwischenwirbelprothese zu fixieren.

Bevorzugt ist das Verankerungselement in einer Verankerungsausnehmung der Prothesenplatte angeordnet und durch ein Betätigungselement zumindest teilweise aus der Verankerungsausnehmung der Prothesenplatte herausbewegbar. Das Betätigungselement kann dabei ebenfalls an der Zwischenwirbelprothese angeordnet oder als separates Werkzeug, welches anschließend aus dem Zwischenwirbelraum entfernt wird, ausgebildet sein.

Vorzugsweise weist das Betätigungselement eine Zwangsfläche auf, auf welcher bei Bewegung des Betätigungselementes eine Auflagekante des Verankerungselementes läuft, um auf diese Weise die Bewegung des Verankerungselementes oder zumindest eines Teiles davon aus der Aufnahmeausnehmung der Prothesenplatte heraus zu bewirken. Vorzugsweise ist die Zwangsfläche dazu keil- oder kegelförmig ausgebildet.

Eine besonders einfache Ausgestaltung von Verankerungselementen ergibt sich, wenn das Verankerungselement U-förmig ausgebildet ist, wobei ein freies Ende eines der Schenkel des U-förmigen Verankerungselementes an der Zwangsfläche des Betätigungselementes anliegt, welches auf diese Weise bei Bewegung des Betätigungselementes derart über die Zwangsfläche läuft, dass es aus der Verankerungsausnehmung der Prothesenplatte herausgeführt wird.

Gemäß einer besonders bevorzugten Ausführungsform der Erfindung weist das Verankerungselement ein Rastelement auf, welches in der aus der Ausnehmung herausbewegten Position mit einem Rastelement des Betätigungselementes zusammenwirkt, um auf diese Weise zu verhindern, dass nach Einbringen der Zwischenwirbelprothese in den Zwischenwirbelraum und Ausfahren der Verankerungselemente versehentlich die Verankerungselemente in die Verankerungsausnehmung zurückgleiten können und die Gefahr besteht, dass sich die Zwischenwirbelprothese löst.

Das Rastelement des Verankerungselementes ist vorzugsweise als an einem freien Ende eines der Schenkel des U-förmigen Verankerungselementes angeordneter Rastvorsprung und das Rastelement des Betätigungselementes als im Anschluss an die keil-oder kegelförmige Zwangsfläche angeordnete umlaufende Nut ausgebildet, was eine besonders einfache Rastverbindung ermöglicht.

Die Erfindung wird anhand der nachfolgenden Figuren ausführlich erläutert.

### Es zeigen

- Fig. 1a: eine Seitenansicht eines ersten Ausführungsbeispieles einer Zwischenwirbelprothese mit einer ersten Prothesenplatte und einer zweiten Prothesenplatte,
- Fig. 1b: eine weitere Seitenansicht der Zwischenwirbelprothese gemäß Fig. 1a,
- Fig. 1c: eine perspektivische Ansicht der Zwischenwirbelprothese gemäß Fig. 1a,
- Fig. 1d: eine Draufsicht auf die Zwischenwirbelprothese gemäß Fig. 1a,
- Fig. 2a: eine Seitenansicht der zweiten Prothesenplatte der Zwischenwirbelprothese gemäß Fig. 1a,
- Fig. 2b: eine perspektivische Ansicht der Prothesenplatte gemäß Fig. 2a,
- Fig. 3a: eine perspektivische Ansicht der ersten Prothesenplatte der Zwischenwirbelprothese gemäß Fig. 1a,
- Fig. 4a: die beiden Prothesenplatten der Zwischenwirbelprothese gemäß Fig. 1a mit abgenommener erster Prothesenplatte,
- Fig. 4b: die Zwischenwirbelprothese gemäß Fig. 4a mit aufgesetzter erster Prothesenplatte,

- Fig. 4c: die Zwischenwirbelprothese gemäß Fig. 4a in gestauchter Position,
- Fig. 5a: eine gekippte Position der Zwischenwirbelprothese gemäß Fig. 1a,
- Fig. 5b: eine weitere gekippte Position der Zwischenwirbelprothese gemäß Fig. 1a,
- Fig. 6a: die Zwischenwirbelprothese gemäß Fig. 1a mit linear verschobener erster Prothesenplatte,
- Fig. 6b: die Zwischenwirbelprothese gemäß Fig. 1a mit der ersten Prothesenplatte in einer weiteren linear verschobenen Position,
- Fig. 7a: eine weitere gekippte Position der Zwischenwirbelprothese gemäß Fig. 1a,
- Fig. 7b: eine weitere gekippte Position der Zwischenwirbelprothese gemäß Fig. 1a,
- Fig. 8a: eine perspektivische Ansicht der ersten Prothesenplatte der Zwischenwirbelprothese gemäß Fig. 1a,
- Fig. 8b: die erste Prothesenplatte gemäß Fig. 8a in einer weiteren perspektivischen Darstellung,
- Fig. 8c: ein Verankerungselement und ein Betätigungselement in perspektivischer Ansicht,
- Fig. 8d: das Verankerungselement gemäß Fig. 8c mit aufgesetztem Betätigungselement,
- Fig. 8e: die erste Prothesenplatte gemäß Fig. 8a mit eingesetztem Verankerungselement gemäß Fig. 8c,
- Fig. 9a: eine Seitenansicht eines weiteren Ausführungsbeispiels einer zweiten Prothesenplatte mit einem eingesetzten Führungselement,
- Fig. 9b: eine perspektivische Ansicht der zweiten Prothesenplatte gemäß Fig. 9a,
- Fig. 9c: eine weitere perspektivische Ansicht der zweiten Prothesenplatte gemäß Fig. 9a,
- Fig. 10a: eine perspektivische Ansicht eines zweiten Ausführungsbeispiels einer ersten Prothesenplatte mit eingesetztem Führungselement und der zweiten Prothesenplatte gemäß Figur 9a,
- Fig. 10b: eine Draufsicht auf die Innenseite der ersten Prothesenplatte gemäß Figur 10a,
- Fig. 10c: eine perspektivische Ansicht der ersten Prothesenplatte gemäß Figur 10b,
- Fig. 11: einen Axialschnitt durch die ersten Prothesenplatte gemäß Figur 10c mit eingesetztem Führungselement,
- Fig. 12a: eine Draufsicht auf eine Prothesenplatte mit einer alternativen Ausführungsform von Schlitzen,
- Fig. 12b: eine Draufsicht auf eine Prothesenplatte mit einer weiteren alternativen Ausführungsform von Schlitzen,
- Fig. 12c: eine Draufsicht auf eine Prothesenplatte mit einer weiteren alternativen Ausführungsform von Schlitzen,
- Fig. 12d: eine Draufsicht auf eine Prothesenplatte mit einer weiteren alternativen Ausführungsform von Schlitzen,
- Fig. 12e: eine Draufsicht auf eine Prothesenplatte mit einer weiteren alternativen Ausführungsform von Schlitzen,
- Fig. 12f: eine Draufsicht auf eine Prothesenplatte mit einer weiteren alternativen Ausführungsform von Schlitzen,
- Fig. 12g: eine Draufsicht auf eine Prothesenplatte mit einer weiteren alternativen Ausführungsform von Schlitzen,
- Fig. 12h: eine Draufsicht auf eine Prothesenplatte mit einer weiteren alternativen Ausführungsform von Schlitzen,
- Fig. 12i: eine Draufsicht auf eine Prothesenplatte mit einer weiteren alternativen Ausführungsform von Schlitzen,
- Fig. 12j: eine Draufsicht auf eine Prothesenplatte mit einer weiteren alternativen Ausführungsform von Schlitzen,
- Fig. 12k: eine Draufsicht auf eine Prothesenplatte mit einer weiteren alternativen Ausführungsform von Schlitzen,
- Fig. 121: eine Draufsicht auf eine Prothesenplatte mit einer weiteren alternativen Ausführungsform von Schlitzen,

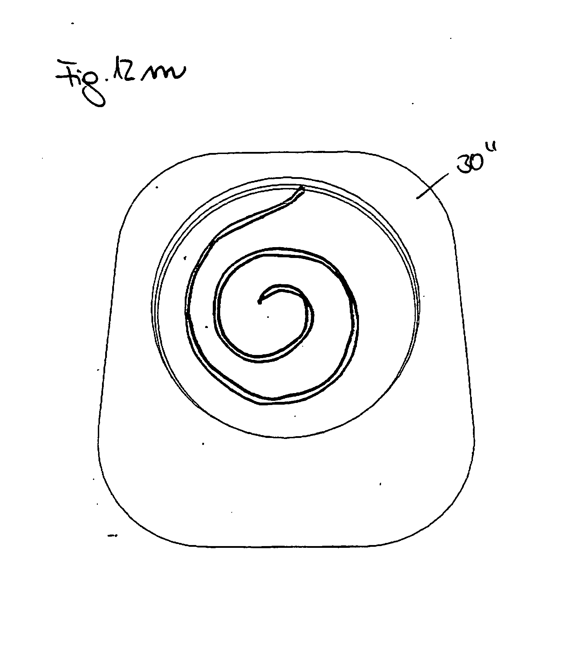

- Fig. 12m: eine Draufsicht auf eine Prothesenplatte mit einer weiteren alternativen Ausführungsform von Schlitzen,

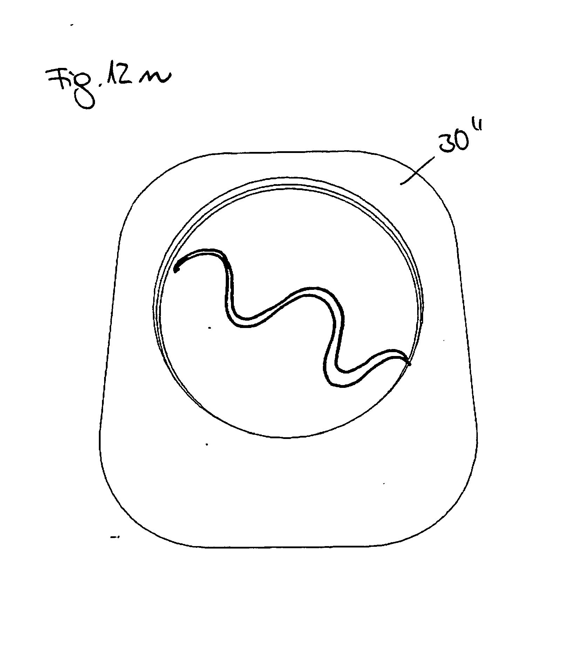

- Fig. 12n: eine Draufsicht auf eine Prothesenplatte mit einer weiteren alternativen Ausführungsform von Schlitzen,

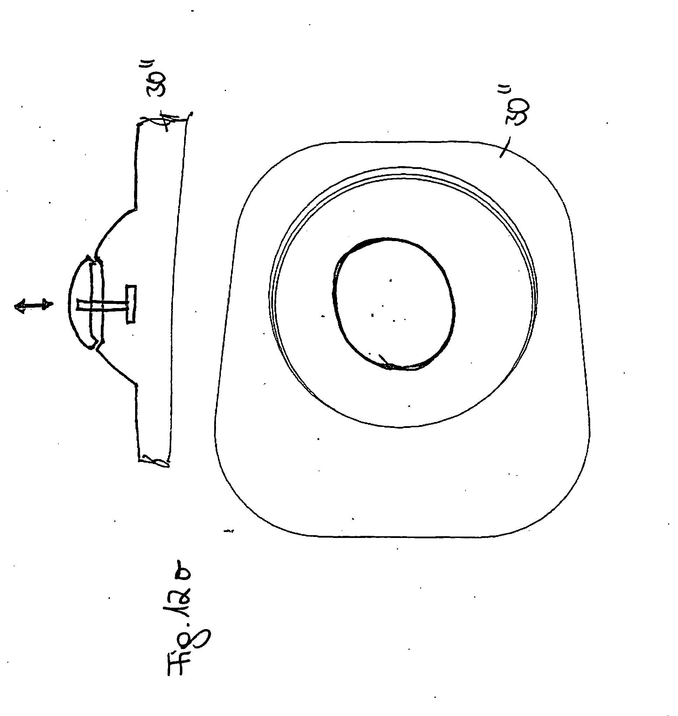

- Fig. 12o: eine Draufsicht auf eine Prothesenplatte mit einer weiteren alternativen Ausführungsform von Schlitzen,

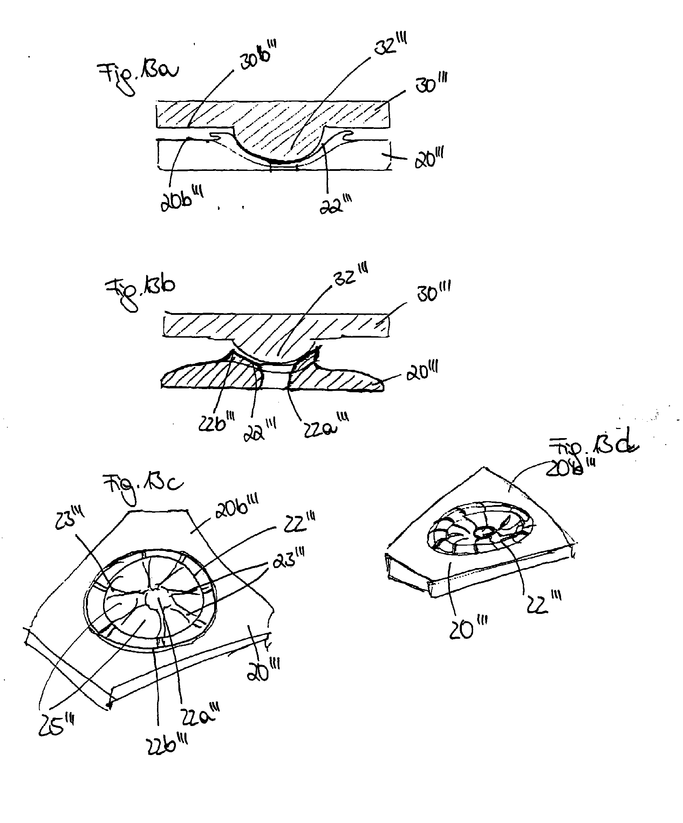

- Fig. 13a: einen Axialschnitt durch ein weiteres Ausführungsbeispiel einer Zwischenwirbelprothese mit einer ersten und einer zweiten Prothesenplatte,
- Fig. 13b: einen Axialschnitt durch ein geringfügig abgewandeltes Ausführungsbeispiel der Zwischenwirbelprothese gemäß Figur 13a,

- Figur 13c: eine perspektivische Ansicht der ersten Prothesenplatte der Zwischenwirbelprothese gemäß Figur 13a und
- Figur 13d: eine weitere perspektivische Ansicht der ersten Prothesenplatte der Zwischenwirbelprothese gemäß Figur 13a.

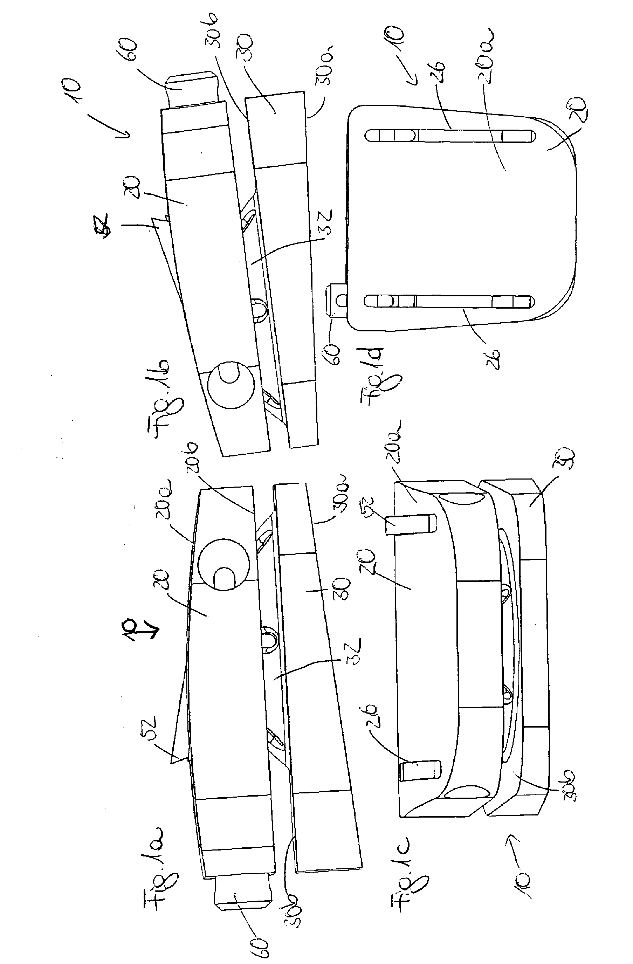

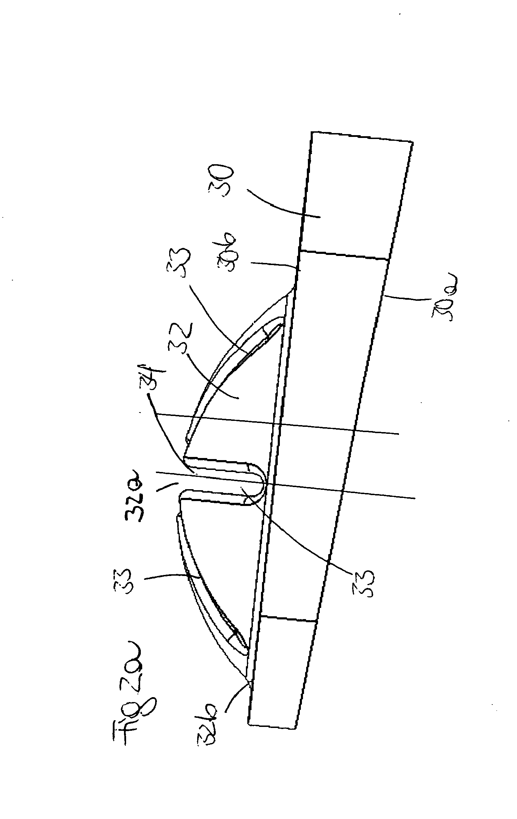

Die Figuren 1-8 zeigen verschiedene Ansichten, teilweise auch nur von Einzelteilen, eines ersten Ausführungsbeispieles einer Zwischenwirbelprothese 10. In allen Figuren sind gleiche Teile mit gleichen Bezugsziffern bezeichnet, wobei zur besseren Übersicht nicht in sämtlichen Figuren sämtliche Bezugsziffern angegeben sind.

Die Zwischenwirbelprothese 10 weist eine erste Prothesenplatte 20 und eine zweite Prothesenplatte 30 auf. Die erste Prothesenplatte 20 weist eine dem benachbarten Wirbel zugewandte Seite 20a und eine der zweiten Prothesenplatte 30 zugewandte Seite 20b auf, während die zweite Prothesenplatte 30 eine dem benachbarten Wirbel zugewandte Seite 30a und eine der ersten Prothesenplatte 20 zugewandte Seite 30b aufweist. Wird die Zwischenwirbelprothese 10 in einen Zwischenraum zwischen zwei benachbarten Wirbeln eingeführt, liegen somit die Seiten 20a, 30a an den benachbarten Wirbeln an, wobei die Zwischenwirbelprothese eine defekte Bandscheibe, die zuvor entfernt wurde, ersetzt.

Sowohl die erste Prothesenplatte 20 als auch die zweite Prothesenplatte 30 sind aus PEEK (Polyetheretherketon) gefertigt.

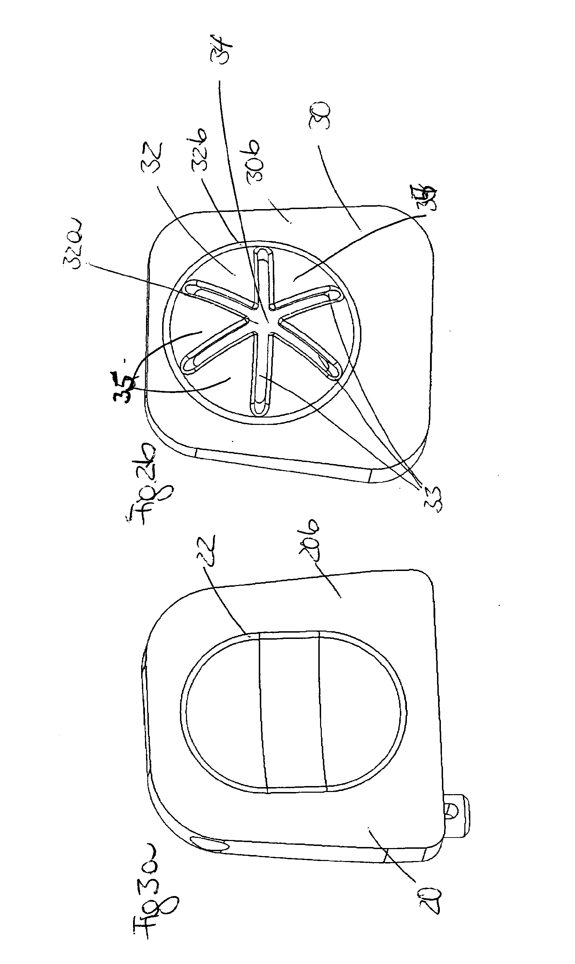

Die erste Prothesenplatte 20 weist auf ihrer der zweiten Prothesenplatte 30 zugewandten Seite 20b, wie insbesondere in Fig. 3a zu sehen, eine konkave Ausnehmung 22 auf, welche etwa ellipsoidschalensegmentförmig ausgebildet ist, sodass die in der Seite 20a liegende Begrenzung der Ausnehmung 22 etwa oval oder elliptisch ausgebildet ist. Die zweite Prothesenplatte 30 weist auf ihrer der ersten Prothesenplatte 20 zugewandten Seite 30b einen konvexen Vorsprung 32 auf, welcher etwa kugelsegmentförmig ausgebildet ist. Der Vorsprung 32 weist einen höchsten Punkt der gekrümmten Oberfläche, einen Pol 32a, auf. Der Vorsprung 32 weist zudem einen etwa kreisförmigen Rand 32b auf, entlang welchem der Vorsprung 32 an die Seite 30b angrenzt. Der Vorsprung 32 ist hohl ausgebildet und weist ausgehend von dem Pol 32a sechs Schlitze 33 im gleichen Winkelabstand zueinander auf, wodurch sechs etwa dreieckig ausgebildete und nach innen gekrümmte federnde Elemente 35 ausgebildet werden.

Der Vorsprung 32 ist in sagittaler Richtung nach posterior versetzt von der Mitte der zweiten Prothesenplatte 30 angeordnet, um das natürliche Rotationszentrum der zu ersetzenden Bandscheibe zu simulieren. Ist die Zwischenwirbelprothese 10 zusammengesetzt, greift der konvexe Vorsprung 32 in die konkave Ausnehmung 22 ein, wodurch ein Kugelgelenk gebildet wird, da der Vorsprung 32 in der Ausnehmung 22 verdreh- und verkippbar und zusätzlich aufgrund der langgestreckten konkaven Ausnehmung 22 in sagittaler Richtung linear bewegbar angeordnet ist.

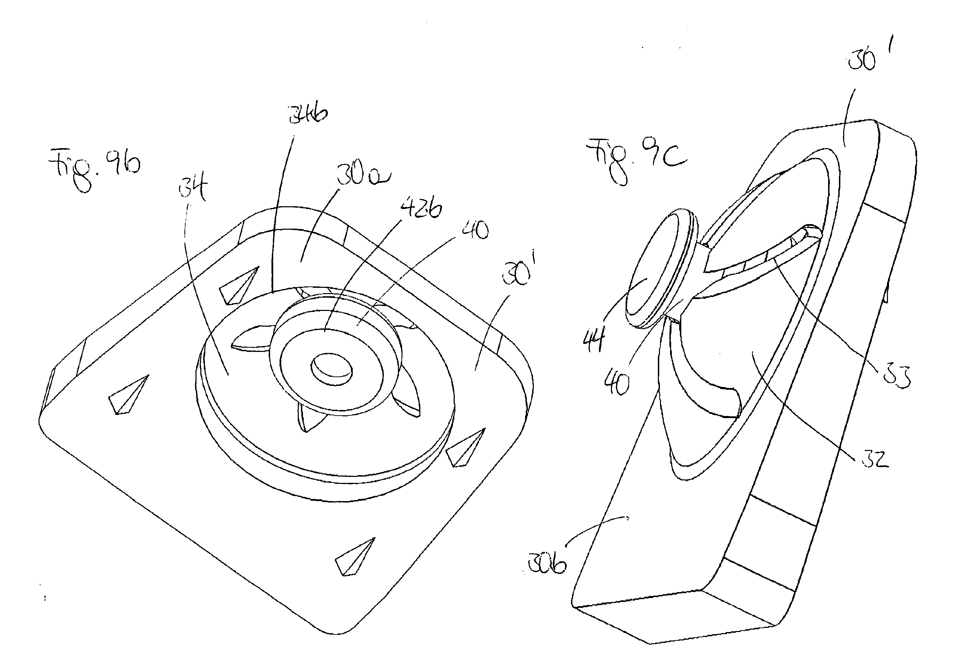

Die federnden Elemente 35 des Vorsprungs 32 erlauben es, die beiden Prothesenplatten 20, 30 in axialer Richtung gegeneinander zusammenzudrücken, sodass bei hoher Belastung der Wirbelsäule eine Dämpfung der Bewegung erfolgt. Die Schlitze 33 sind dabei vorzugsweise derart ausgefertigt, dass sich die federnden Elemente 35 bei hoher Belastung derart nach innen biegen, dass die federnden Elemente 35 mit ihren Rändern aneinander liegen, bis insbesondere ein kugelsegmentförmiger Vorsprung 32 größeren Radius gebildet ist. Dadurch wird gewährleistet, dass auch bei hoher Belastung der Vorsprung 32 möglichst reibungsarm in der Ausnehmung 22 bewegbar ist. Besonders bevorzugt sind die Schlitze 33 schräg zur Oberfläche des Vorsprungs 32 in den Vorsprung eingeschnitten. Dies ist insbesondere in Figur 9c, welche nachfolgend noch näher beschrieben wird, deutlich erkennbar. Durch die schräg eingeschnittenen Schlitze 33 wird nicht nur ermöglicht, dass die Ränder der federnden Elemente 35 bei hoher Belastung aneinander liegen, sondern es wird weiterhin ermöglicht, dass die Ränder benachbarter federnder Elemente 35 in radialer Richtung des Vorsprungs 32 aufeinander liegen, wodurch die Stabilität des Vorsprungs 32 auch bei hoher Belastung gewährleistet ist.

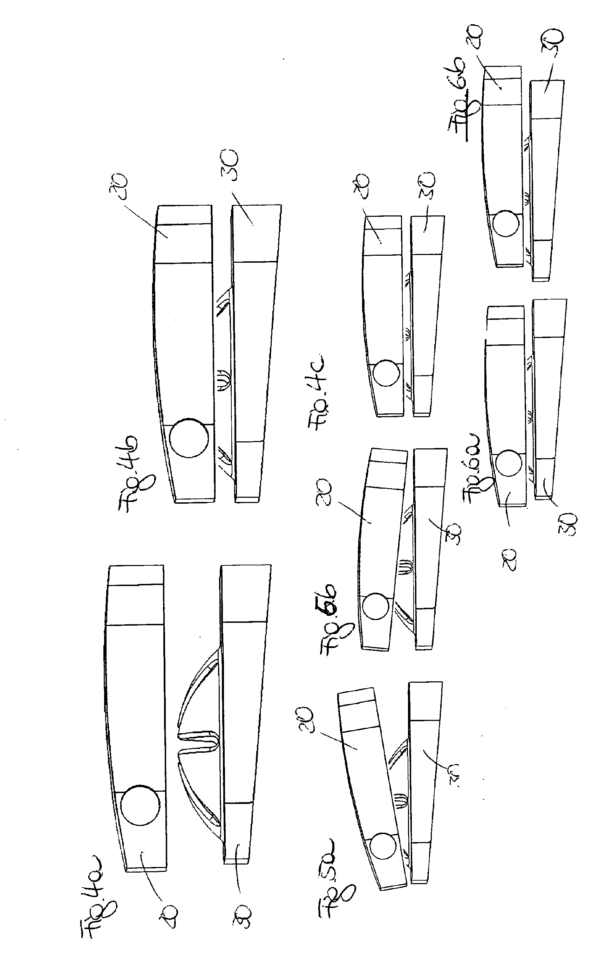

Die Figuren 4-7 zeigen verschiedene Positionen der beiden Prothesenplatten 20, 30 gegeneinander. Figur 4a zeit die Zwischenwirbelprothese 10 mit in axialer Richtung abgehobener erster Prothesenplatte 20. Figur 4b zeigt die Zwischenwirbelprothese 10 mit auf die zweite Prothesenplatte 30 aufgesetzter erster Prothesenplatte 20 im unbelasteten Zustand, während Figur 4c die Zwischenwirbelprothese 10 mit auf die zweite Prothesenplatte 30 aufgesetzter erster Prothesenplatte 20 unter hoher Belastung zeigt, wobei die beiden Prothesenplatten 20, 30 etwa um 0,5 mm zusammengedrückt werden.

Die Figuren 5a und 5b zeigen eine Seitenansicht der Zwischenwirbelprothese 10 aus transversaler Richtung, wobei die erste Prothesenplatte 20 in den beiden Figuren in die maximale Position nach vorne bzw. nach hinten gekippt dargestellt ist. Es ist mit der Zwischenwirbelprothese 10 eine Flexions- und Extensionsbewegung von etwa +/- 8° möglich.

In den Figuren 6a und 6b ist die Zwischenwirbelprothese 10 in einer Seitenansicht aus transversaler Richtung dargestellt, wobei die erste Prothesenplatte 20 gegenüber der zweiten Prothesenplatte 30 in die maximalen Positionen nach vorne bzw. nach hinten linear verschoben dargestellt ist. Diese lineare Verschiebung wird durch die langgestreckte konkave Ausnehmung 22 ermöglicht, wobei eine Linearbewegung um bis zu 1,5 mm möglich ist.

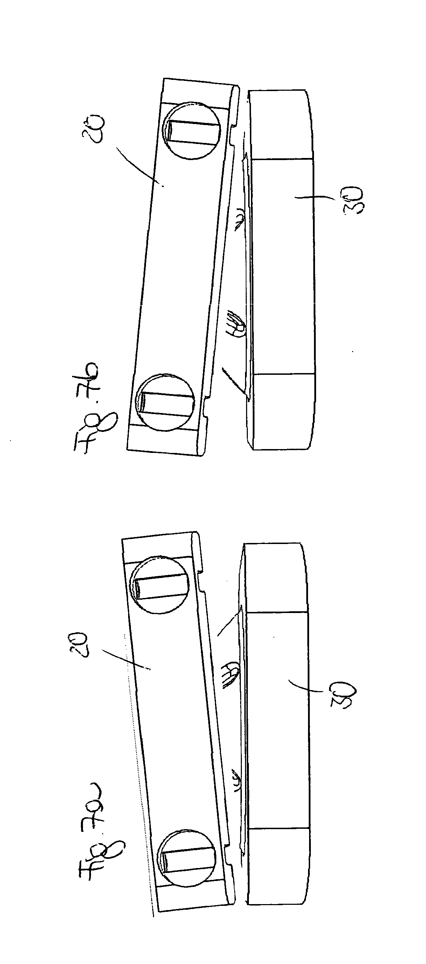

Die Figuren 7a und 7b zeigen die Zwischenwirbelprothese 10 in einer Seitenansicht aus sagittaler Richtung, wobei die erste Prothesenplatte 20 gegenüber der zweiten Prothesenplatte 30 in den beiden maximalen seitlichen Neigungen dargestellt ist. Mit der Zwischenwirbelprothese 10 sind Neigungen bis zu +/- 8° möglich. Zudem ermöglicht die Zwischenwirbelprothese 10 einen Rotationsradius um die vertikale Achse der Zwischenwirbelprothese 10 zwischen +/- 2°.

Um eine möglichst schnelle und dauerhafte biologische Integration der Zwischenwirbelprothese 10 in den Körper des Patienten zu ermöglich, werden die Prothesenplatten 20, 30 zumindest teilweise, insbesondere die den benachbarten Wirbeln zugewandten Seiten 20a, 20b, mit einer Oberflächenbeschichtung, insbesondere einer osteokonduktiven oder osteoinduktiven Beschichtungen, versehen. Beispielsweise kann eine Hydroxylapathit-Beschichtung durch ein Plasmasprayverfahren aufgebracht werden. Alternativ kann auch Tricalciumphosphat als Beschichtungswerkstoff verwendet werden. Derartige Beschichtungen weisen kein Metall, insbesondere kein Titan auf, so dass diese Beschichtungen eine artefaktfreie MRT-Aufnahme ermöglichen.

An den Prothesenplatten 20, 30 können auf den den benachbarten Wirbeln zugewandten Seiten 20a, 30a Verankerungselemente in Form von vorstehenden Zähnen oder sonstigen Vorsprüngen oder Aufrauhungen angebracht sein, um die Prothesenplatten 20, 30 an den benachbarten Wirbeln zu fixieren.

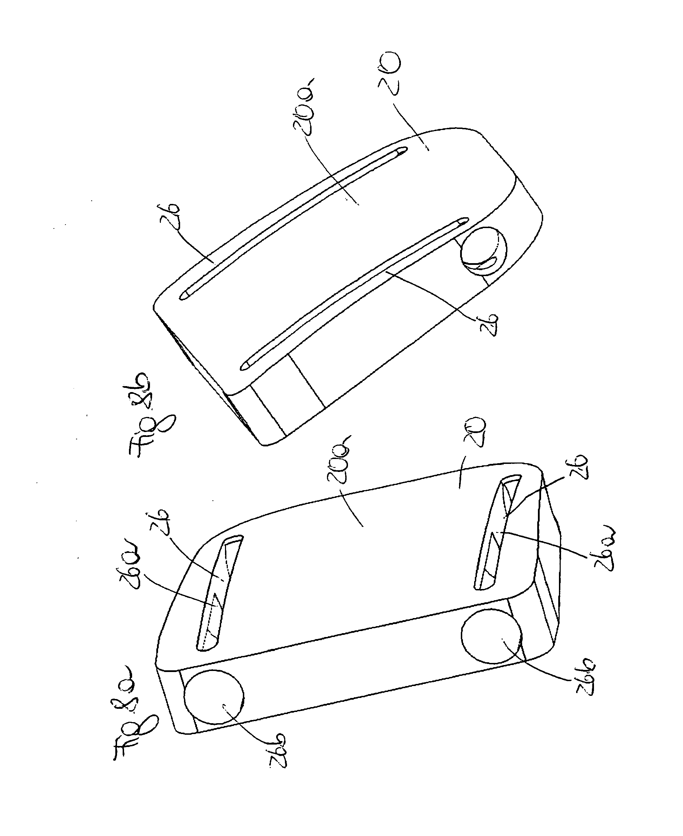

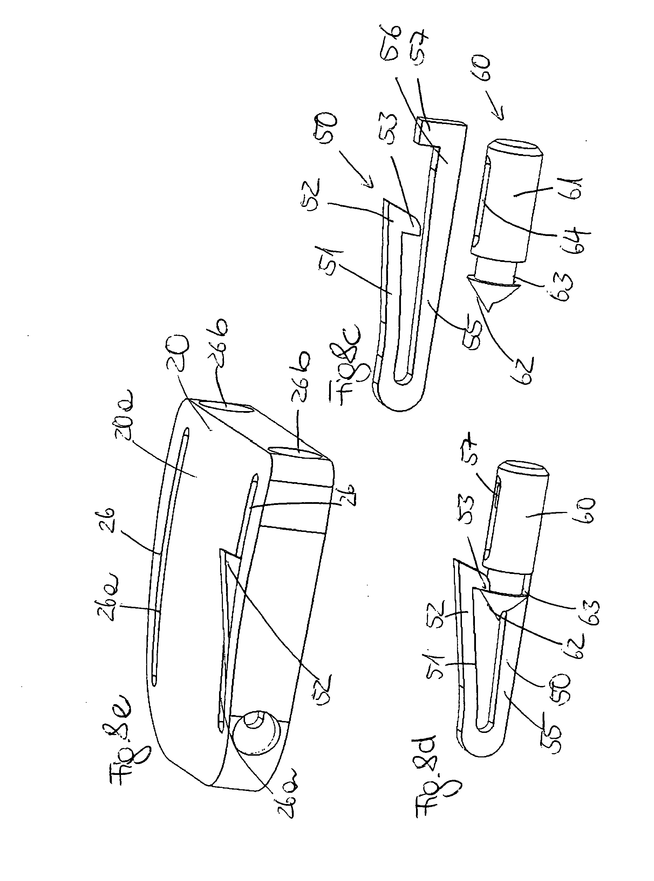

In den Figuren 8a-8e ist ein bevorzugter Verankerungsmechanismus zur Verankerung einer der beiden Prothesenplatten, vorliegend der ersten Prothesenplatte 20, in dem benachbarten Wirbel dargestellt. Die erste Prothesenplatte 20 weist in ihrer dem benachbarten Wirbel zugewandten Seite 20a zwei parallel zueinander in sagittaler Richtung verlaufende Verankerungsausnehmung 26 auf, in welchen jeweils ein Verankerungselement 50 angeordnet werden kann. Das Verankerungselement 50 ist etwa U-förmig ausgebildet mit einem ersten Schenkel 51 und einem zweiten Schenkel 55, wobei der zweite Schenkel 55 länger ausgebildet ist als der erste Schenkel 51. Der zweite Schenkel 55 weist ein freies Ende 56 auf, an welchem ein nach innen abgewinkelter Haltevorsprung 57 angeordnet ist.

Das Verankerungselement 50 kann mit Hilfe eines Betätigungselementes 60 in der Verankerungsausnehmung 26 versenkt und zumindest teilweise aus dieser herausbewegt werden. Das Betätigungselement 60 weist einen etwa zylindrischen Grundkörper 61 auf, an welchem an einem Ende eine Zwangsfläche 62 angeordnet ist, welche etwa kegelförmig ausgebildet ist. Im Anschluss an die Zwangsfläche 62 weist der Grundkörper 61 eine umlaufende Nut 63 geringeren Durchmessers als der Durchmesser des Grundkörpers 61 auf. In dem Grundkörper 61 ist diametral ein Querschlitz 64 angeordnet. Der Querschlitz 64 ist entlang eines Radius durch die Nut 63 und die Zwangsfläche 62 bis zum durch die Zwangsfläche 62 gebildeten Ende des Betätigungselementes 60 fortgesetzt. Auf diese Weise wird es ermöglicht, das Betätigungselement 60 auf das freie Ende 56 des zweiten Schenkels 55 des Verankerungselementes 50 derart aufzusetzen, dass der Haltevorsprung 57 in den Querschlitz 64 eingreift und das Betätigungselement 60 entlang des freien Endes 56 des zweiten Schenkels 55 des Verankerungselementes 50 über den Fortsatz des Querschlitzes 64 in der Nut 63 und der Zwangsfläche 62 linear geführt bewegbar ist.

Der erste Schenkel 51 des Verankerungselementes 50 weist ein freies Ende 52 auf, an welchem sich nach innen ein Rastvorsprung 53 erstreckt. Ist das Betätigungselement 60 auf dem zweiten Schenkel 55 am weitesten in Richtung des freien Endes 56 des zweiten Schenkels 55 bewegt, liegt der Rastvorsprung 53 vor der Zwangsfläche 62. Wird das Betätigungselement 60 entlang des zweiten Schenkels 55 in Richtung auf den die beiden Schenkel 51, 55 verbindenden Bereich des U-förmigen Verankerungselementes 50 geschoben, bewirkt die keil- oder kegelförmige Zwangsfläche 62 des Betätigungselementes 60, dass der Rastvorsprung 53 mit einer Auflagekante auf ihm entlang laufend von dem zweiten Schenkel 55 wegbewegt und das U-förmige Verankerungselement 50 aufgebogen wird.

Das Verankerungselement 50 wird in einem ersten Teilbereich 26a der Verankerungsausnehmung 26 der ersten Prothesenplatte 20 derart angeordnet, dass die Ebene des U des Verankerungselementes 50 senkrecht zur Seite 20a bzw. 20b verläuft. Das Verankerungselement 50 ist derart dimensioniert, dass es im nicht aufgebogenen Zustand in der Verankerungsausnehmung 26 vollständig eingebettet ist.

Der etwa schlitzförmige und zur Seite 20a offene Teilbereich 26a der Verankerungsausnehmung 26 setzt sich in sagittaler Richtung nach anterior versetzt in einem zylindrischen Teilberich 26b fort, in welchem das Betätigungselement 60 linear verschiebbar geführt angeordnet werden kann. Wird das Betätigungselement 60 entlang des zweiten Schenkels 55 in die erste Prothesenplatte 20 hineingedrückt, bewirkt die Zwangsfläche 62 ein Aufbiegen des ersten Schenkels 51 des Verankerungselementes 50, sodass das freie Ende 52 des ersten Schenkels 51 über die Seite 20a hinaussteht (vgl. Fig. 8e). Diese hervorstehenden Bereiche des Verankerungselementes 50 werden in den benachbarten Wirbelkörper hineingedrückt und bewirken auf diese Weise eine Verankerung der Prothesenplatte 20 an dem benachbarten Wirbel. Wird jedoch die Zwischenwirbelprothese 10 in den Zwischenwirbelraum eingeführt, sind die Verankerungselemente 50 zunächst in der Verankerungsausnehmung 26 versenkt, sodass das Einführen der Zwischenwirbelprothese 10 in den Zwischenwirbelraum vereinfacht wird, da der Zwischenwirbelraum weniger aufgespreizt werden muss.

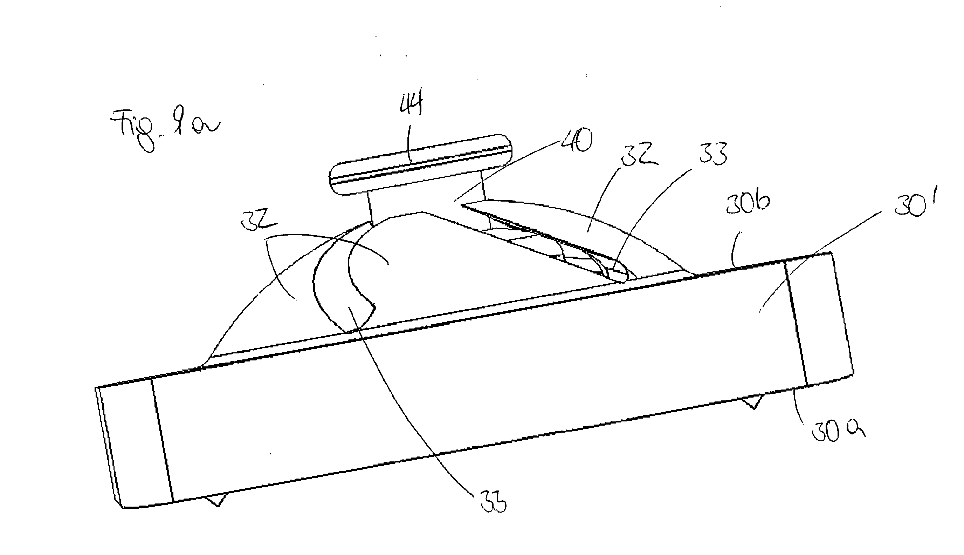

Um zu verhindern, dass bei unphysiologischen Bewegungen oder Schleudertraumen die beiden Prothesenplatten 20, 30 außer Eingriff geraten, können statt der Prothesenplatten 20, 30 auch geringfügig modifizierte Prothesenplatten 20', 30' verwendet werden, zwischen welchen ein Führungselement 40 angeordnet wird, welches nachfolgend anhand der Figuren 9 bis 11 beschrieben wird.

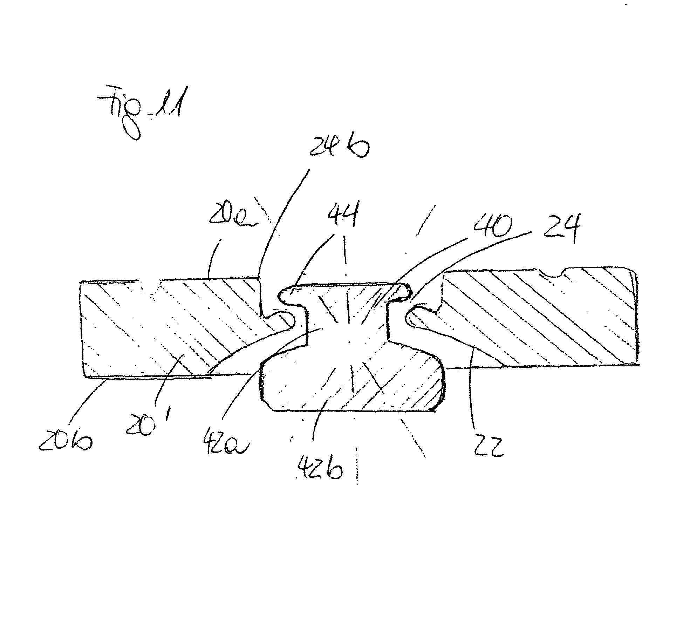

Das Führungselement 40 weist einen Grundkörper 42 auf, an dessen einen Ende ein Kopf 44 angeordnet ist. Der Kopf 44 kann zylindrisch, insbesondere mit abgerundeten Kanten, halbkugelförmig oder kugelförmig ausgebildet sein. Der Grundkörper 42 weist einen stiftartigen oder zylindrischen Bereich 42a und einen Verankerungsbereich 42b auf, wie insbesondere in Figur 11 erkennbar. Dabei ist der Grundkörper 42 insbesondere zweiteilig ausgebildet, wobei der Verankerungsbereich 42b lösbar an dem Bereich 42a angeordnet ist.

Der Grundkörper 42 des Führungselementes 40 ist in einer Führungsausnehmung 34 in der zweiten Prothesenplatte 30' angeordnet, wobei sich die Führungsausnehmung 34 im Wesentlichen in radialer Richtung durch den Vorsprung 32 erstreckt und im Wesentlichen koaxial zur Rotationsachse der Zwischenwirbelprothese 10 zwischen den federnden Elementen 35 und in dem hohlen Vorsprung 32 bis zur Seite 30a der Prothesenplatte 30' verläuft. Wie in Figur 9b dargestellt ist, ist die Führungsausnehmung 34 in der Seite 30a mit einer Öffnung 34b ausgestattet, so dass die Führungsausnehmung in axialer Richtung durch die Prothesenplatte 30' verläuft. Die Führungsausnehmung 34 fixiert dabei den Grundkörper 42 in horizontaler und vertikaler Richtung. Dies erfolgt im wesentlichen dadurch, dass der Verankerungsbereich 42b des Grundkörpers 40 an der Innenfläche der Seite 30a der Prothesenplatte 30' fixiert wird, wenn die Öffnung 34b der Führungsausnehmung durch eine nicht dargestellte Abschlussplatte verschlossen wird, so dass weder eine horizontale noch eine vertikale Bewegung des Führungselements 40 relativ zu der zweiten Prothesenplatte 30' möglich ist.

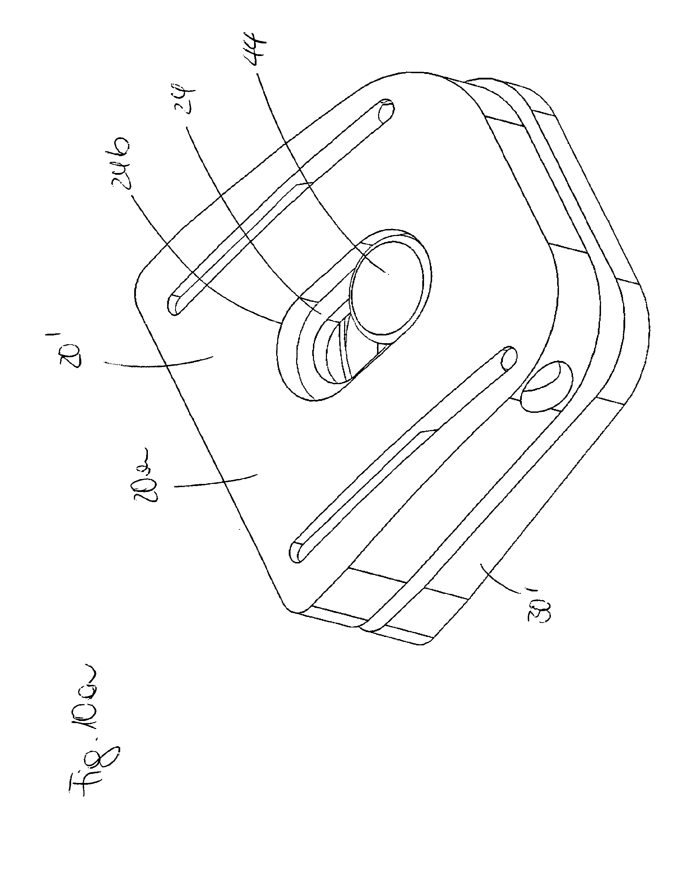

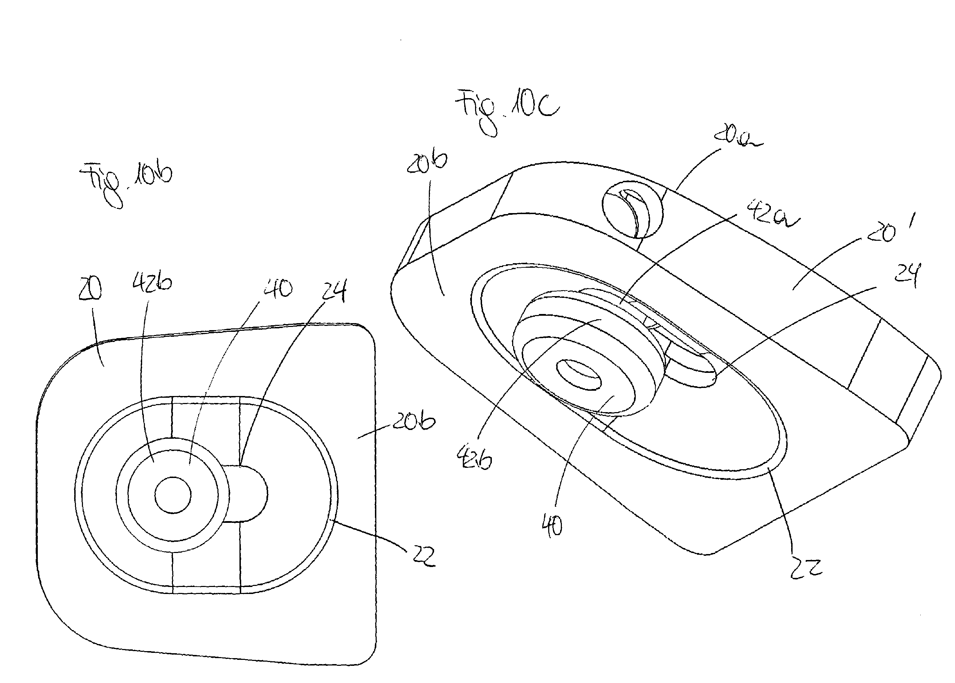

Die Figuren 10a, 10b und 10c zeigen die erste Prothesenplatte 20', welche sich von der zuvor beschriebenen ersten Prothesenplatte 20 dadurch unterscheidet, dass in der Ausnehmung 22 eine zusätzliche Führungsausnehmung 24 angeordnet ist. In der Artikulationsfläche der Ausnehmung 22 bildet die Führungsausnehmung 24 eine etwa ovale oder elliptische Fläche und erstreckt sich in die erste Prothesenplatte 20' als hinterschnittene Ausnehmung. Wie insbesondere der Figur 10a und Figur 11 zu entnehmen ist, erstreckt sich die Führungsausnehmung 24 in axialer Richtung durch die Prothesenplatte 20' und weist eine Öffnung 24b in der Seite 20a auf.

Der Kopf 44 des Führungselementes 40 wird derart in die Führungsausnehmung 24 eingebracht, dass er in der hinterschnittenen Führungsausnehmung 24 in horizontaler und vertikaler Richtung bewegt werden kann. Bei Verdrehung, Verkippung oder linearer Bewegung des Vorsprungs 32 in der Ausnehmung 22 bewegt sich der Kopf 44 in der hinterschnittenen Führungsausnehmung 24 der Ausnehmung 22 der ersten Prothesenplatte 20' und behindert somit die Bewegung kaum. Bei großer Belastung kann sich das Führungselement 40 in axialer Richtung in der Führungsausnehmung 24 bewegen, wobei jedoch durch die Länge des Grundkörpers 42 des Führungselementes 40 ein Anschlag für eine maximale Stauchung der Zwischenwirbelprothese 10 gebildet wird. Das Führungselement 40 verhindert jedoch, dass sich die erste Prothesenplatte 20' bei zu großen Relativbewegungen vollständig von der zweiten Prothesenplatte 30' lösen kann.

Zum Einsetzen des Führungselements 40 in die zweite Prothesenplatte 30' wird somit das Führungselement 40 in zwei Teile zerlegt. Von der Seite 20a der ersten Prothesenplatte 20' wird der Kopf 44 mit dem daran angeordneten Bereich 42a voran durch die Öffnung 24b in die Führungsausnehmung 24 eingeführt, bis das freie Ende des Bereichs 42a in der konkaven Ausnehmung 22 angeordnet ist. Anschließend wird der Verankerungsbereich 42b an dem Bereich 42a angeordnet. Anschließend werden sowohl auf die Öffnung 24b der Führungsausnehmung 24 der ersten Prothesenplatte 20' als auch auf die Öffnung 34b der Führungsausnehmung 34 der zweiten Prothesenplatte 30' Abschlussplatten aufgesetzt, um die Öffnungen 24b, 34b zu verschließen.

Die Schlitze 33 des zweiten Ausführungsbeispiels der Prothesenplatte 30' sind ausgehend von dem Pol 32a des Vorsprungs 32 in Umfangswinkelrichtung gekrümmt zum Rand 32b verlaufend angeordnet.

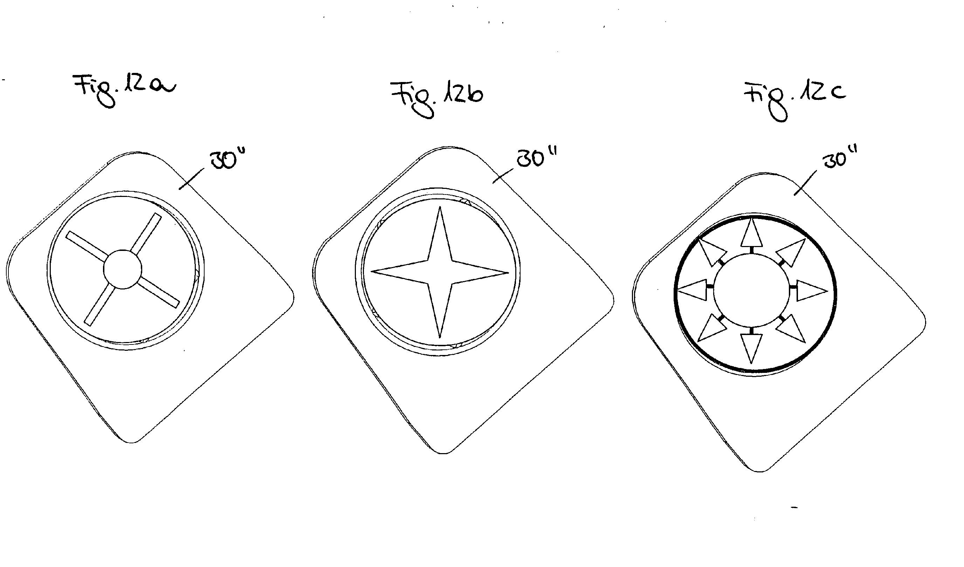

In den Figuren 12a bis 12o ist eine Prothesenplatte 30" mit einem konvexen Vorsprung mit verschiedenen Ausführungsformen von Schlitzen, welche die Federwirkung des konvexen Vorsprungs begünstigen, dargestellt. Diese Form von Schlitzen kann alternativ auch in einer konkaven Ausnehmung angeordnet werden.

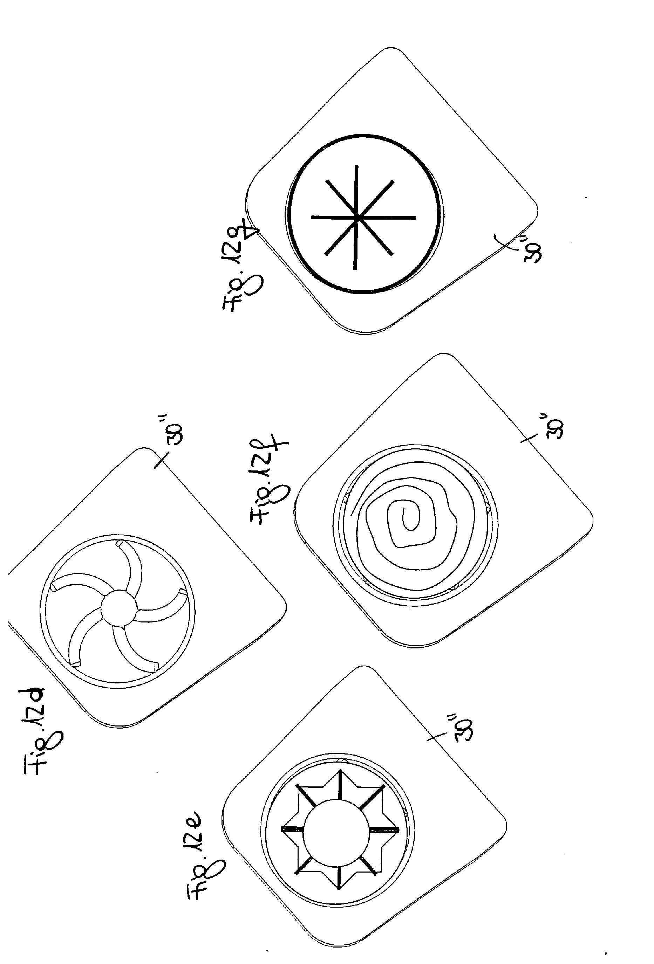

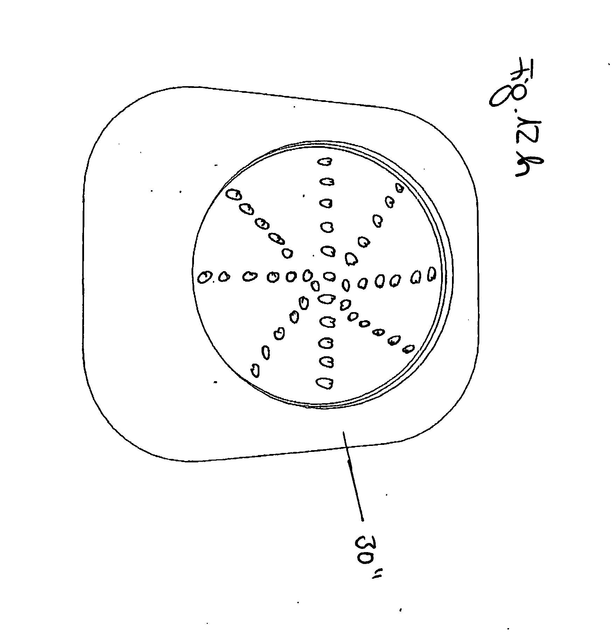

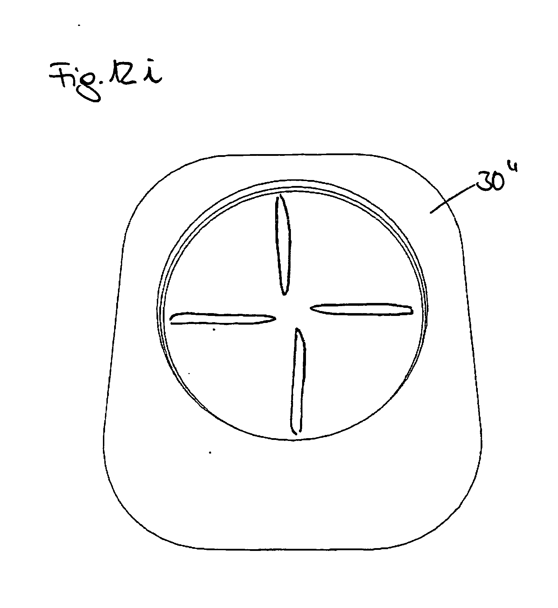

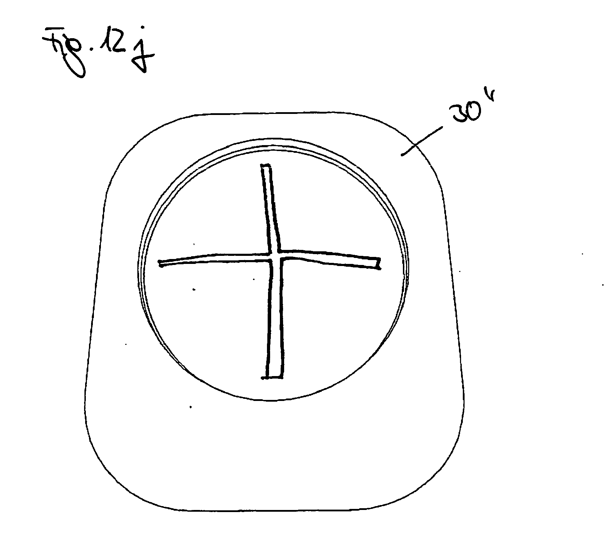

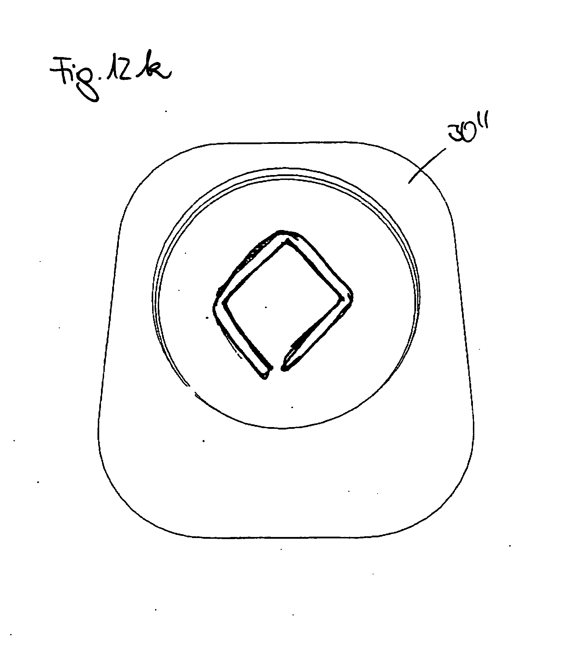

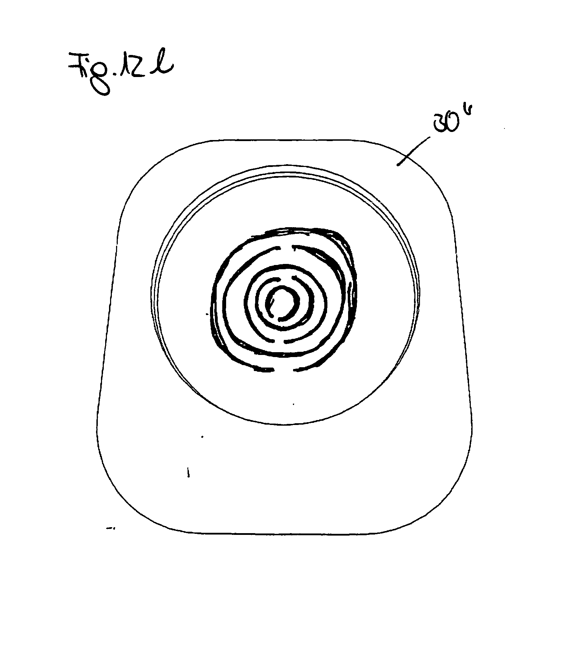

Die verschiedenen Ausführungsformen weisen beispielsweise ausgehend vom Pol des konvexen Vorsprungs in Richtung des Randes verlaufende Schlitze auf (vgl. Fig. 12a,12b, 12d, 12g, 12j), welche sich teilweise verjüngen (vgl. Fig. 12b) oder gekrümmt verlaufen (vgl. Fig. 12d). Fig. 12c zeigt einen konvexen Vorsprung mit einer größeren Öffnung um den Pol und angrenzend etwa dreieckig ausgebildete Schlitze. In dem Ausführungsbeispiel gemäß Figur 12e ist eine etwa sternförmige Ausnehmung als Schlitz vorgesehen. Die Figuren 12f und 12m zeigen einen spiralförmig gekrümmten Schlitz, während in Figur 12l mehrere konzentrisch verlaufende Schlitze dargestellt sind, wobei die Schlitze jedoch keinen geschlossenen Ring bilden. In Figur 12h sind mehrere Lochreihen in dem konvexen Vorsprung angeordnet. Figur 12i zeigt vier zum Rand des konvexen Vorsprungs verlaufende Schlitze, die jedoch am Pol nicht miteinander in Verbindung stehen wie beispielsweise die Schlitze in Figur 12j. Figur 12k zeigt einen um den Pol umlaufenden, etwa quadratisch ausgebildeten Schlitz, welcher jedoch ebenfalls keinen geschlossenen Ring bildet. Figur 12n zeigt einen in mehreren Krümmungen, insbesondere wellenförmig verlaufenden Schlitz, welcher sich von einem Rand des konvexen Vorsprungs über den Pol bis etwa zum gegenüberliegenden Rand des Vorsprungs erstreckt.

In den Ausführungsbeispielen der Figuren 12a bis 12n sind die Schlitze jeweils so ausgebildet, dass der konvexe Vorsprung einteilig ausgebildet ist. Figur 12o zeigt ein Beispiel für einen zweiteilig ausgebildeten konvexen Vorsprung, bei welchem durch einen um den Pol umlaufenden Schlitz der Pol abgetrennt und über ein zwischen der Polkappe und dem verbleibenden Rest des konvexen Vorsprungs, insbesondere der Prothesenplatte 30", ein Element angeordnet ist, welches eine Relativbewegung der Polkappe zur Prothesenplatte in axialer Richtung zulässt.

In den Figuren 13a bis 13d ist ein Ausführungsbeispiel einer Zwischenwirbelprothese mit einer ersten Prothesenplatte 20''', welche auf einer Seite 20b''' eine konkave Ausnehmung 22''' aufweist, und einer zweiten Prothesenplatte 30''', welche auf einer der ersten Prothesenplatte 20''' zugewandten Seite 30b''' einen konvexen, etwa kugelsegmentförmigen Vorsprung 32''' aufweist, welcher in die konkave Ausnehmung 22''' eingreift, dargestellt, bei welchem die konkave Ausnehmung 22''' wenigstens ein federndes Element 25''' aufweist. Die konkave Ausnehmung 22''' ist im wesentlichen kugelschalenförmig ausgebildet und weist einen Pol 22a''' und einen Rand 22b''' auf.

Die Figuren 13a und 13b zeigen jeweils einen Axialschnitt durch die Zwischenwirbelprothese, wobei geringfügige Unterschiede darin bestehen, dass bei dem Ausführungsbeispiel gemäß Figur 13a der Rand 22b''' der Ausnehmung 22''' nach außen gebogen ist und einen hinterschnittenen umlaufenden Rand 22b''' ausbildet, während der Rand 22b''' der Ausnehmung 22''' des Ausführungsbeispiels in Figur 13b nicht nach außen gebogen ist.

Die Figuren 13c und 13d zeigen zwei perspektivische Ansichten der ersten Prothesenplatte 20''' der Zwischenwirbelprothese gemäß Figur 13a, in welchen erkennbar ist, dass in der Ausnehmung 22''' die federnden Elemente 25''' dadurch gebildet werden, dass ausgehend von dem Pol 22a''' der Ausnehmung 22''' mehrere Schlitze zum Rand 22b''' verlaufen. Die Schlitze 23''' können auch andere Ausgestaltungen annehmen, beispielsweise wie in den Figuren 12a bis 12o dargestellt. Der konvexe Vorsprung 32''' der zweiten Prothesenplatte 30''' kann massiv oder hohl ausgebildet sein und kann bei hoher Belastung der Zwischenwirbelprothese in axialer Richtung gegen die Federkraft der federnden Elemente 25''' in die Ausnehmung 22''' tiefer hineingedrückt werden.

### Bezugszeichenliste

- 10: Zwischenwirbelprothese
- 20: erste Prothesenplatte
- 20': erste Prothesenplatte
- 20''': erste Prothesenplatte
- 20a: Seite
- 20b: Seite
- 20b''': Seite
- 22: Ausnehmung
- 22b''': Ausnehmung
- 22a"': Pol
- 22b''': Rand
- 23''': Schlitz
- 24: Führungsausnehmung
- 24b: Öffnung
- 25''': federndes Element
- 26: Verankerungsausnehmung
- 26a: Teilbereich
- 26b: Teilbereich
- 30: zweite Prothesenplatte
- 30': zweite Prothesenplatte
- 30'': zweite Prothesenplatte
- 30''': zweite Prothesenplatte
- 30a: Seite
- 30b: Seite
- 30b''': Seite
- 32: Vorsprung
- 32''': Vorsprung
- 32a: Pol
- 32b: Rand
- 33: Schlitz
- 34: Führungsausnehmung
- 34b: Öffnung
- 35: federndes Element
- 40: Führungselement
- 42: Grundkörper
- 42a: Bereich
- 42b: Verankerungsbereich
- 44: Kopf
- 50: Verankerungselement
- 51: erster Schenkel
- 52: freies Ende
- 53: Rastvorsprung
- 55: zweiter Schenkel
- 56: freies Ende
- 57: Haltevorsprung
- 60: Betätigungselement
- 61: Grundkörper
- 62: Zwangsfläche
- 63: Nut
- 64: Querschlitz

## Patentansprüche

1. Zwischenwirbelprothese (10) mit einer ersten Prothesenplatte (20) und einer zweiten Prothesenplatte (30), wobei die erste Prothesenplatte (20) auf ihrer der zweiten Prothesenplatte (30) zugewandten Seite (20b) eine konkave Ausnehmung (22) aufweist, in welche ein auf der der ersten Prothesenplatte (10) zugewandten Seite (30b) der zweiten Prothesenplatte (30) angeordneter konvexer Vorsprung (32) eingreift,
**dadurch gekennzeichnet, dass** der konvexe Vorsprung (32) und/oder die konkave Ausnehmung zumindest ein federndes Element (35) aufweist.

2. Zwischenwirbelprothese nach einem der vorhergehenden Ansprüche,
**dadurch gekennzeichnet, dass** der konvexe Vorsprung (32) und/oder die konkave Ausnehmung hohl ausgebildet ist.

3. Zwischenwirbelprothese nach einem der vorhergehenden Ansprüche,
**dadurch gekennzeichnet, dass** der konvexe Vorsprung (32) und/oder die konkave Ausnehmung wenigstens einen Schlitz (33) aufweist.

4. Zwischenwirbelprothese nach einem der vorhergehenden Ansprüche,
**dadurch gekennzeichnet, dass** der wenigstens eine Schlitz (33) schräg zur Oberfläche des konvexen Vorsprungs (32) oder der konkaven Ausnehmung eingeschnitten ist.

5. Zwischenwirbelprothese nach einem der vorhergehenden Ansprüche,
**dadurch gekennzeichnet, dass** der konvexe Vorsprung (32) und/oder die konkave Ausnehmung ausgehend von seinem Pol (32a) mehrere in Richtung des Rands (32b) des konvexen Vorsprungs (32) verlaufende Schlitze (33) aufweist.

6. Zwischenwirbelprothese nach einem der vorhergehenden Ansprüche,
**dadurch gekennzeichnet, dass** die Ausnehmung kugelschalensegmentförmig oder ellipsoidschalensegmentförmig ausgebildet ist.

7. Zwischenwirbelprothese nach einem der vorhergehenden Ansprüche,
**dadurch gekennzeichnet, dass** der Vorsprung (32) kugelsegmentförmig ausgebildet ist.

8. Zwischenwirbelprothese nach einem der vorhergehenden Ansprüche,
**dadurch gekennzeichnet, dass** der Vorsprung (32) in sagittaler Richtung von der Mitte der Prothesenplatte (30) nach posterior versetzt angeordnet ist.

9. Zwischenwirbelprothese nach einem der vorhergehenden Ansprüche,
**dadurch gekennzeichnet, dass** zwischen der ersten Prothesenplatte (20) und der zweiten Prothesenplatte (30) ein Führungselement (40) angeordnet ist, welches einen Grundkörper (42) und einen an einem Ende des Grundkörpers (42) angeordneten Kopf (44) aufweist,
wobei der Grundkörper (42) in eine Führungsausnehmung (34) in der zweiten Prothesenplatte (30) und der Kopf (44) in eine Führungsausnehmung (24) in der ersten Prothesenplatte (20) eingreift.

10. Zwischenwirbelprothese nach Anspruch 9,
**dadurch gekennzeichnet, dass** die Führungsausnehmung (34) der zweiten Prothesenplatte (30) in dem Vorsprung (32), insbesondere zwischen den federnden Elementen (35), angeordnet ist.

11. Zwischenwirbelprothese nach einem der Ansprüche 9 bis 10,
**dadurch gekennzeichnet, dass** die Führungsausnehmung (34) der zweiten Prothesenplatte (30) das Führungselement (40) in horizontaler und vertikaler Richtung relativ zur zweiten Prothesenplatte (30) fixiert.

12. Zwischenwirbelprothese nach einem der Ansprüche 9 bis 11,
**dadurch gekennzeichnet, dass** die Führungsausnehmung (24) der ersten Prothesenplatte (20) in der Ausnehmung (22) angeordnet ist.

13. Zwischenwirbelprothese nach einem der Ansprüche 9 bis 12,
**dadurch gekennzeichnet, dass** die Führungsausnehmung (24) der ersten Prothesenplatte (20) eine Bewegung des Führungselements (40) in horizontaler und vertikaler Richtung zulässt.

14. Zwischenwirbelprothese nach einem der Ansprüche 9 bis 13,
**dadurch gekennzeichnet, dass** die Führungsausnehmung (24) der ersten Prothesenplatte (20) oval oder elliptisch ausgebildet ist, wobei die Längsachse der Führungsausnehmung (24) in sagittaler Richtung verläuft.

15. Zwischenwirbelprothese nach einem der vorhergehenden Ansprüche,
**dadurch gekennzeichnet, dass** die erste Prothesenplatte (20) und/oder die zweite Prothesenplatte (30) aus PEEK gefertigt ist.

16. Zwischenwirbelprothese nach einem der vorhergehenden Ansprüche,
**dadurch gekennzeichnet, dass** die dem Wirbel zugewandte Seite (20a, 30a) der ersten Prothesenplatte (20) und/oder der zweiten Prothesenplatte (30) eine osteokonduktive und/oder eine osteoinduktive Beschichtung aufweist.
